(19) **Europäisches Patentamt**
**European Patent Office**
**Office européen des brevets**

(11) **EP 1 330 542 B1**

(12) **FASCICULE DE BREVET EUROPEEN**

(45) Date de publication et mention
de la délivrance du brevet:
**07.07.2010 Bulletin 2010/27**

(51) Int Cl.:
*C12Q 1/68* (2006.01)   *C12Q 1/70* (2006.01)

(21) Numéro de dépôt: **01974442.4**

(86) Numéro de dépôt international:
**PCT/FR2001/003077**

(22) Date de dépôt: **05.10.2001**

(87) Numéro de publication internationale:
**WO 2002/029096 (11.04.2002 Gazette 2002/15)**

(54) **METHODE RAPIDE DE DETECTION D'ORGANISMES DANS UN ECHANTILLON**

METHODE ZUM SCHNELLNACHWEIS VON ORGANISMEN IN EINER PROBE

FAST METHOD FOR DETECTING ORGANISMS IN A SAMPLE

(84) Etats contractants désignés:
**AT BE CH CY DE DK ES FI FR GB GR IE IT LI LU MC NL PT SE TR**

(30) Priorité: **05.10.2000 FR 0012717**

(43) Date de publication de la demande:
**30.07.2003 Bulletin 2003/31**

(73) Titulaire: **Skuld-Tech S.A.R.L.**
**34090 Montpellier (FR)**

(72) Inventeurs:
• **QUERE, Ronan**
**29800 La Forest Landerneau (FR)**
• **COMMES MAERTEN, Thérèse**
**F-34570 Vailhauques (FR)**
• **MARTI, Jacques**
**F-34980 Saint Clement de Riviere (FR)**
• **PIQUEMAL, David**
**F-30520 Saint Martin de Valgagues (FR)**

(74) Mandataire: **de Zeeuw, Johan Diederick**
**Murgitroyd & Company,**
**Immeuble Atlantis**
**55, Allée Pierre Ziller,**
**Sophia Antipolis**
**06560 Valbonne (FR)**

(56) Documents cités:
**EP-A- 0 785 255 WO-A-93/09245**
**WO-A-98/28444**

• **ATLAS R M ET AL: "DETECTING BACTERIAL PATHOGENS IN ENVIRONMENTAL WATER SAMPLES BY USING PCR AND GENE PROBES" US, SAN DIEGO, ACADEMIC PRESS, 1989, pages 399-406, XP000363601**
• **ANTHONY R M ET AL: "Rapid Diagnosis of Bacteremia by Universal Amplification of 23S Ribosomal DNA Followed by Hybridization to an Oligonucleotide Array" JOURNAL OF CLINICAL MICROBIOLOGY, WASHINGTON, DC, US, vol. 38, no. 2, février 2000 (2000-02), pages 781-788, XP002176098 ISSN: 0095-1137**
• **GUSCHIN D Y ET AL: "OLIGONUCLEOTIDE MICROCHIPS AS GENOSENSORS FOR DETERMINATIVE AND ENVIRONMENTAL STUDIES IN MICROBIOLOGY" APPLIED AND ENVIRONMENTAL MICROBIOLOGY, US, WASHINGTON, DC, vol. 63, no. 6, 1 juin 1997 (1997-06-01), pages 2397-2402, XP002064989 ISSN: 0099-2240**
• **LO C -F ET AL: "SPECIFIC GENOMIC DNA FRAGMENT ANALYSIS OF DIFFERENT GEOGRAPHICAL CLINICAL SAMPLES OF SHRIMP WHITE SPOT SYNDROME VIRUS" DISEASES OF AQUATIC ORGANISMS, XX, XX, vol. 35, no. 3, 26 février 1999 (1999-02-26), pages 175-185, XP000878518**
• **LO C-F ET AL: "Detection of baculovirus associated with white spot syndrome (WSBV) in penaeid shrimps using polymerase chain reaction" DISEASES OF AQUATIC ORGANISMS, XX, XX, XP002074932**

EP 1 330 542 B1

- KUHNERT P ET AL: "DETECTION SYSTEM FOR ESCHERICHIA COLI-SPECIFIC VIRULENCE GENES: ABSENCE OF VIRULENCE DETERMINANTS IN B AND C STRAINS" APPLIED AND ENVIRONMENTAL MICROBIOLOGY, WASHINGTON,DC, US, vol. 63, no. 2, février 1997 (1997-02), pages 703-709, XP000980041 ISSN: 0099-2240
- BERTUCCI F ET AL: "Sensitivity issues in DNA array-based expression measurements and performance of nylon microarrays for small samples." HUMAN MOLECULAR GENETICS. ENGLAND SEP 1999, vol. 8, no. 9, septembre 1999 (1999-09), pages 1715-1722, XP002227849 ISSN: 0964-6906
- CHEN JEREMY J W ET AL: "Profiling expression patterns and isolating differentially expressed genes by cDNA microarray system with colorimetry detection" GENOMICS, ACADEMIC PRESS, SAN DIEGO, US, vol. 51, no. 3, 1 août 1998 (1998-08-01), pages 313-324, XP002159705 ISSN: 0888-7543
- KIM C K ET AL: "DEVELOPMENT OF A POLYMERASE CHAIN REACTION (PCR) PROCEDURE FOR THE DETECTION OF BACULOVIRUS ASSOCIATED WITH WHITE SPOT SYNDROME (WSBV) IN PENAEID SHRIMP" JOURNAL OF FISH DISEASES,OXFORD,GB, vol. 21, no. 1, janvier 1998 (1998-01), pages 11-17, XP000997469
- HOELTKE H J ET AL: "SENSITIVE CHEMILUMINESCENT DETECTION OF DIGOXIGENIN-LABELED NUCLEIC ACIDS: A FAST AND SIMPLE PROTOCOL AND ITS APPLICATIONS" BIOTECHNIQUES,US,EATON PUBLISHING, NATICK, vol. 12, no. 1, 1992, pages 104-106,108,11, XP000770649 ISSN: 0736-6205
- WANG Q ET AL: "IDENTIFICATION OF GENOMIC VARIATIONS AMONG GEOGRAPHIC ISOLATES OF WHITE SPOT SYNDROME VIRUS USING RESTRICTION ANALYSIS AND SOUTHERN BLOT HYBRIDIZATION" DISEASES OF AQUATIC ORGANISMS,XX,XX, vol. 43, 21 décembre 2000 (2000-12-21), pages 175-181, XP001004618

Remarques:

Le dossier contient des informations techniques présentées postérieurement au dépôt de la demande et ne figurant pas dans le présent fascicule.

**Description**

La présente invention concerne les méthodes de détection et plus particulièrement une méthode de détection d'organismes dans un échantillon.

Notre société est de plus en plus sensible aux problèmes de contamination des produits naturels et plus particulièrement des aliments. Les cas de listériose et de salmonellose d'origine alimentaire en sont des illustrations très actuelles. A ces problèmes de santé publique majeurs se greffent des cas de contaminations plus ponctuels, principalement dues à un manque d'hygiène ou à une mauvaise conservation, notamment dans les restaurants ou les cantines. Quelle que soit la nature de la contamination, il est aujourd'hui primordial, d'une part, de pouvoir détecter rapidement la présence de l'agent pathogène et d'autre part de déterminer l'origine de la contamination. Ainsi, la traçabilité des produits naturels tout au long de la chaîne de distribution est devenue un souci majeur tant pour les pouvoirs publics que pour les sociétés qui souhaitent ne plus être tenues pour responsables des problèmes de contamination.

En effet, un système de traçabilité permet non seulement de pouvoir détecter rapidement la présence d'un agent infectieux, mais également de déterminer à quel maillon de la chaîne de production est intervenue la contamination afin de l'enrayer.

Afin de mettre en évidence la présence d'agents pathogènes, des contrôles qualité sont mis en place tout au long de la chaîne de production. Ces contrôles sont généralement basés sur des analyses microbiologiques des matières premières ou des produits finis lorsqu'il s'agit des produits de confection. Des contrôles de l'environnement de production peuvent également être réalisés. Relativement efficaces pour des produits alimentaires confectionnés, ces contrôles n'ont que peu d'efficacité pour des produits de l'agriculture, qu'il s'agisse de l'élevage ou de la culture. En effet, la nécessité d'obtenir des résultats rapides est souvent peu compatible avec le temps nécessaire à la recherche de microorganismes.

Les maladies telles que les parasitoses, les bactérioses, les viroses ou les mycoses peuvent être prévenues par des techniques vaccinales et sont donc contrôlables.

Cependant, chez certains animaux d'élevage, les traitements préventifs sont, à ce jour, trop complexes ou coûteux. En outre, ils ne sont pas envisageables chez les invertébrés car ce type d'animaux ne possède pas de système de défense autorisant la vaccination.

Par exemple, les viroses sont fréquemment observées dans des élevages de crustacés et elles peuvent avoir des effets dévastateurs puisqu'elles se propagent rapidement et à grande échelle.

L'élimination des sujets infectés est donc souvent la seule solution pour éradiquer les contaminations dans certains élevages.

Cependant, depuis quelques années, le développement de la génétique et des outils de biologie moléculaire ont permis l'élaboration de nouveaux outils de diagnostic. Ces nouvelles techniques sont principalement basées sur la détection des acides nucléiques des agents pathogènes et en particulier de leur ADN.

En effet, l'ADN est la seule macromolécule biologique qui puisse être amplifiée *in vitro* de façon exponentielle, par réaction en chaîne de l'ADN polymérase (PCR), conférant aux méthodes de détection basées sur cette réaction une sensibilité inégalée.

L'ADN étant présent dans tous les organismes, il est primordial de s'assurer de la spécificité du fragment amplifié par PCR (amplicon). Dans les méthodes classiques, celle-ci est assurée par la spécificité des amorces de PCR, oligonucléotides synthétiques qui, en s'hybridant de part et d'autre du segment d'ADN recherché, vont garantir son identification. Néanmoins, ce seul critère n'est pas suffisant. En général, une électrophorèse de l'amplicon permet à la fois de le visualiser et d'estimer sa taille, apportant ainsi un critère supplémentaire. Cette méthode, couramment employée dans les laboratoires de recherche, est également proposée dans certains kits de diagnostic. Cependant, elle est souvent trop peu sensible et demande une intervention manuelle difficilement automatisable.

L'hybridation d'une sonde spécifique s'associant sélectivement à l'ADN recherché est souvent nécessaire pour préciser le diagnostic.

Une première méthode est l'hybridation in situ. Il s'agit d'une méthode précise et très utile pour localiser les cibles cellulaires, par exemple d'un virus. Elle consiste à utiliser les tissus fixés d'un animal pris dans la cire, coupés en fines sections et placés sur une lame microscopique. La lame portant le tissu et la sonde de détection sont ensuite chauffées, de façon à séparer les doubles brins d'ADN. Le tissu est ensuite mis en contact avec la sonde de détection. Il s'agit d'une sonde ADN, marquée avec une molécule spécifique telle que la digoxigénine (DIG). La sonde marquée à la DIG s'hybride (se lie) très spécifiquement à la cible ADN de l'organisme recherché si celui-ci est présent dans l'échantillon. Des anticorps spécifiques de la DIG couplés à une enzyme sont ajoutés à la lame. Les anticorps conjugués se lient à la DIG et lorsque le substrat de l'enzyme est ajouté, un changement de couleur se produit. Ce précipité coloré est alors visible dans les tissus spécifiquement infectés par l'organisme. Le précipité est visible au microscope.

Cette méthode possède différents inconvénients. Elle consiste tout d'abord en un procédé long, demandant plusieurs jours de préparation du tissu, afin de pouvoir le couper. Elle nécessite également de pouvoir accéder à un laboratoire d'histologie sophistiqué, dans lequel il est possible de trouver les outils nécessaires à la préparation et à l'observation de sections de tissus. Il existe de plus un risque important de faux négatifs dans le cas où le niveau de contamination du produit ou de l'organisme est faible.

La deuxième méthode basée sur l'utilisation de sondes génomiques est la méthode "Dot Blot". Il s'agit d'une méthode qui comporte la même procédure de base que l'hybridation In Situ (Khandjian, 1987). Cependant, la détection ne se fait pas sur des tissus fixés, mais sur des tissus macérés ou un échantillon de sang, appliqué sur une membrane.

La "cible", correspondant à l'échantillon d'ADN à tester, est immobilisée en premier sur la membrane, avant de placer ladite membrane dans la solution d'hybridation. La "sonde", correspondant à l'ADN de l'organisme recherché, est marquée, puis est placée dans la solution d'hybridation avec ladite membrane. Ce milieu réactionnel est ensuite placé à incubation.

Avant leur application sur la membrane, les échantillons sont tout d'abord chauffés pour séparer les brins d'ADN de l'organisme recherché, si celui-ci est présent. De même, la sonde d'ADN, marquée à la DIG, doit être chauffée pour séparer les brins d'ADN, avant d'être ajoutée sur la membrane. Le système de révélation à la DIG permet de visualiser une tache sombre, indicatrice de l'infection des échantillons. L'intensité de la tache est proportionnelle à la quantité d'organismes présents dans l'échantillon. En effet, plus la tache est sombre et plus l'infection est importante.

Cette méthode possède également des inconvénients. L'inconvénient majeur est qu'il est parfois difficile de distinguer les réactions positives du bruit de fond. Il est donc nécessaire d'introduire des contrôles appropriés sur la membrane test. Toutefois, l'interprétation des résultats reste subjective. De plus, cette méthode n'est pas assez sensible pour pouvoir détecter les faibles niveaux de contamination. Enfin, les sondes n'étant pas immobilisées mais en suspension dans la solution d'hybridation, il faut utiliser des sondes spécifiques à un seul organisme pathogène car rien ne permet de distinguer les sondes entre elles. On ne peut donc détecter qu'un seul organisme pathogène à la fois.

De nouveaux outils, appelés "puces à ADN", ont été développés dans les cinq dernières années. Les "puces à ADN" sont des supports de plastique, de verre, de silicium ou autre, d'une taille avoisinant le centimètre carré, sur lesquels sont greffés ou synthétisés des sondes d'ADN complémentaires du produit recherché d'une longueur de 0,5 à 2 kilobases (kb), ou de courts oligonucléotides, avec lesquels peuvent s'hybrider des séquences d'ADN que l'on cherche à identifier. Grâce à des procédés robotisés, des dizaines de milliers de ces sondes sont disposées, sous forme de "spots", le long d'un réseau régulier gravé à la surface de la puce. Chaque "spot" contient de nombreux exemplaires d'une séquence d'ADN correspondant à un gène unique. Les sondes de la puce sont ensuite hybridées avec des échantillons, c'est à dire l'ensemble des ADN complémentaires (ADNc) correspondant aux ARN messagers extraits de tissus d'origine biologique. Ces échantillons sont marqués, généralement à l'aide d'un fluorochrome. Après lavage, les spots où s'est produite l'hybridation peuvent être révélés par le marquage fluorescent.

Les mesures les plus courantes sont effectuées afin d'évaluer l'expression différentielle de gènes de deux échantillons (M. Schena et al. (1995). Science, 270, 467). Elles sont réalisées par hybridation compétitive en utilisant un marquage par fluorescence en deux couleurs.

La lecture optique par des faisceaux laser capables d'exciter les fluorochromes permet de récolter les données qui seront ensuite traitées par un logiciel permettant d'obtenir une image où est représenté un ensemble de taches colorées d'intensité variable et correspondant aux ADNc des échantillons hybridés aux sondes.

Les "puces à ADN" portent aujourd'hui les noms d'oligonucleotide chips (Affymetrix), de macroarrays (Gress, T.M., Hoheisel, J.D., Lennon, G.G., Zehetner, G. ; Lehrach, H. (1992) - Mamm. Genome, 3, 609) et (Piétu G., Alibert, A., Guichard, V., Lamy, B., Bois, F., et al. (1996) - Genome Research, 6, 492) et de microarrays (DeRisi, J.L., Vishwanath, R.Y. and Brown, P.O. (1997). Science, 278, 680) et (Wodicka, L., Dong, H., Mittmann, M., Ho, M.H., and Lockhart, D.J. (1997). Nature Biotechnology, 15, 1359) selon la taille des dépôts. Les macroarrays représentent des puces de quelques dizaines de sondes par cm2 contrairement aux microarrays ou aux oligonucleotide chips qui contiennent plusieurs milliers de sondes au cm2 .

Les Macroarrays, issus de travaux de l'équipe de B. Jordan publiés en 1995 (Technique de Multiple Messenger Assay (MMA) (C. Nguyen, D. Rocha, S. Granjeaud, M. Baldit, K. Bernard, P. Naquet, and B. R. Jordan, "Differential gene expression in the murine thymus assayed by quantitative hybridization of arrayed cDNA clones," Genomics, vol. 29, pp. 207-216, 1995), concernent des "membranes à haute densité" : des clones d'ADNc (issus d'une banque) ou leurs produits de PCR sont disposés régulièrement sur des membranes de Nylon, lesquelles sont hybridées avec des sondes complexes radioactives produites par rétrotranscription d'une préparation d'ARN messager ou total. Après hybridation, l'ensemble des données est acquis de manière quantitative, généralement par un système à écran phosphore, et le niveau d'expression de chacun des gènes représentés est ainsi mesuré (plus précisément l'abondance relative de chacun des ARN messagers dans le mélange de départ).

Plusieurs fabricants commercialisent des membranes prêtes à l'emploi portant quelques centaines ou quelques milliers de gènes. Sous sa forme la plus répandue, cette technique utilise des membranes d'assez grandes dimensions, avec une densité de quelques dizaines de dépôts par cm2 et un espacement entre les spots supérieur à un millimètre, compatible avec une détection par des sondes marquées à la radioactivité telles que des sondes marquées au phosphore 32 ($^{32}$P) ou au phosphore 33 ($^{33}$P).

Les microarrays, quant à eux, comportent quelques milliers de gènes représentés par des produits de PCR déposés sur une lame de verre traitée. L'hybridation est effectuée avec une sonde (ADNc) complexe obtenue par rétrotranscription du mélange d'ARN en présence de nucléotides substitués autorisant une détection ultérieure (directe ou non) par fluorescence. L'acquisition des résultats est généralement effectuée par un système de balayage laser équipé d'une optique confocale, les signaux étant mesurés par un photomultiplicateur.

Un autre système consiste à synthétiser directement les sondes sur un support de silice. Il s'agit du procédé de synthèse in situ d'oligonucléotides (adressage photochimique) (G. McGall, J. Labadie, P. Brock, G. Wallraff, T. Nguyen, and W. Hinsberg, "Light-directed synthesis of high-density oligonucleotide arrays using semiconductor photoresists," Proc Natl Acad Sci U S A, vol. 93, pp. 13555-60, 1996) exploité par la société Affymetrix. L'hybridation de la sonde et de la cible est suivie par fluorescence.

Différents types de marquage sont actuellement proposés:

- le radiomarquage par des méthodes classiques au phosphore 32 ou 33. La lecture de l'hybridation obtenue est saisie par autoradiographie ou grâce à un appareil tel que le PhosphoImager (Molecular Dynamics) qui permet une quantification précise des signaux,
- la fluorescence qui permet d'atteindre un seuil de détection très bas par l'utilisation, par exemple, du microscope à confocale. De plus, de part la multitude de fluorochromes disponibles sur le marché, un large choix s'offre aux équipes de recherche afin d'affiner la lecture des signaux émis,
- la détection colorimétrique (enzymatique).

Les puces à ADN possèdent toutefois plusieurs inconvénients majeurs. En effet, elles permettent théoriquement l'hybridation simultanée d'un nombre de sondes considérable, permettant ainsi de détecter plusieurs organismes pathogènes différents. Or, réaliser en parallèle des milliers de réactions d'hybridation impliquant des compositions de bases différentes (donc de stabilité différente) représente une difficulté technique importante.

Il est primordial de trouver l'équilibre optimal d'hybridation pour chacun des duplex sonde-cible.

Un autre inconvénient concerne les difficultés de mise en oeuvre de ces techniques. Les puces à ADN à"haute densité"sont d'une très haute sophistication, sont très coûteuses et demandent des équipements pour la lecture et l'interprétation des résultats qui sont lourds, coûteux et complexes à utiliser. La mise au point de nouvelles puces permettant la détection de microorganismes nécessite de multiples compétences dans des domaines aussi variés que l'électronique, la chimie des nucléotides, la micromécanique, la micro-optique, ou encore la micro-chimie, ce qui complique fortement le développement de tels outils.

La spécificité de l'hybridation entre la sonde et la cible (ADN extrait de l'échantillon) est une donnée importante pour ces méthodes de détection. En effet, pour détecter des virus sur un échantillon, il faut que l'hybridation entre la sonde et la cible (ADN extrait de l'échantillon) soit la plus spécifique possible. Moins l'hybridation est spécifique, plus il y aura de faux positifs, plus il y aura de bruit et donc moins le test sera pertinent. Or, dans toutes les méthodes décrites jusqu'alors, l'étape d'hybridation est faite de manière contraignante. C'est à dire que lès produits en présence des sondes sont chauffés à des températures élevées de manière à obtenir une hybridation la plus spécifique possible. Ces hybridations sont suivies de lavages classiques. Ceci impose un matériel spécifique, tels que des fours à hybridation.

Un article paru dans le Journal of Clinical Biology, Washington, DC, Us, Vol. 38, No. 2, février 2000 (2000-2002), pages 781-788 décrit un procédé qui ambitionne de déterminer la présence de bactéries, quelles qu'elles soient, via l'utilisation d'amorces uniques, qualifiées d'universelles, dans la réaction de PCR pour cibler une région du gène de l'ARN ribosomale 23S commune à de nombreuses bactéries. Son intérêt est d'utiliser un seul réactif pour amplifier et détecter les gènes de multiples espèces bactériennes. Il n'y a donc aucune spécificité recherchée dans la technique décrite.

En résumé, les méthodes d'hybridation in situ sont des méthodes complexes, longues, onéreuses et ne permettent pas une détection de niveaux faibles de contamination. Les méthodes de type Dot Blot, sont plus simples mais ne permettent pas la détection de niveaux faibles de contamination ni la détection de plusieurs organismes différents simultanément. L'art antérieur pour pallier ces inconvénients s'est donc orienté vers des méthodes utilisant des « puces à ADN ». Ces méthodes permettent de détecter plusieurs organismes différents et ont une sensibilité accrue. Cependant elles sont complexes, longues, onéreuses et nécessitent des compétences diverses ainsi qu'un matériel très spécifique.

Le but de l'invention est de pallier ces inconvénients en fournissant une méthode rapide de détection de contamination par des organismes, chez un homme, un animal, dans un tissu ou une cellule, facile à mettre en oeuvre, nécessitant peu de moyens techniques, possédant une sensibilité suffisante pour détecter tant les faibles niveaux de contamination que les contaminations importantes, permettant de détecter la présence de plusieurs organismes différents et permettant également de déterminer la quantité d'organismes en présence d'un appareillage simple.

L'invention concerne donc une méthode rapide de détection des ADN d'un ou de plusieurs organismes dans un échantillon consistant à :

- préparer au moins une sonde spécifique dudit ou desdits organismes,

- immobiliser ladite sonde sur un support approprié,
- saturer le support avec des fragments d'ADN ne réagissant pas avec les produits recherchés pour éviter toute interaction non spécifique desdits produits avec le support lui-même,
- extraire l'ADN dudit ou desdits organismes contenus dans l'échantillon provenant d'un produit ou un sujet,
- amplifier ledit ou lesdits ADN du ou des organismes simultanément, afin d'obtenir un ou plusieurs amplicons,
- marquer lesdits amplicons,
- hybrider à température ambiante par dépôt direct l'ADN amplifié et marqué avec la sonde immobilisée sur le support,
- révéler le marquage et,
- examiner directement le résultat, ou obtenir une image numérique de la membrane colorée.

Le terme examen direct signifie que les résultats de la méthode de détection selon la présente invention sont observés à l'oeil nu directement sur la membrane et directement après l'étape de révélation. Afin de quantifier les nuances de couleurs observées, il est possible de numériser l'image de la membrane colorée (à l'aide d'un scanner par exemple). Un simple programme codant les intensités de couleurs permet alors de quantifier la quantité d'organismes présents.

Un autre avantage de l'invention est que l'étape d'hybridation est réalisée de manière non contraignante. La contrainte intervient lors des lavages, en utilisant, au cours d'au moins un de ces lavages, une solution de lavage chauffée à une température comprise entre 40 et 70°C. Dans les techniques de l'art antérieur, c'est l'inverse : l'hybridation se fait à forte stringence et les lavages sont classiques. Par hybridation non contraignante ou à faible stringence, il faut entendre une hybridation à température ambiante (comprise entre 18 et 25°C). Cette nouvelle façon de procéder permet d'augmenter fortement la sensibilité de la méthode selon l'invention. Elle permet également d'éviter l'utilisation de fours à hybridation.

De plus cette méthode permet de procéder à un dépôt direct de l'échantillon sur le support. Ainsi, la réaction d'hybridation se réalise dans le volume délimité par les dimensions du support. En concentrant les réactifs dans un faible volume réactionnel, cela permet de réduire la durée de l'analyse et d'améliorer la sensibilité de la méthode.

Selon un mode de réalisation préférentiel l'amplification des ADN et le marquage desdits amplicons sont effectués simultanément.

L'amplification desdits amplicons peut également être effectuée selon une PCR.

L'invention présente en outre l'avantage de permettre de préparer des sondes différentes. Ceci permet de détecter plusieurs organismes différents mais également de préparer des sondes témoins.

L'immobilisation des sondes sur le support permet en effet de repérer chacune des sondes spécifiques à un organisme déterminé. Au cours de l'étape d'amplification, on procède à l'amplification de chaque ADN recherché à partir d'amorces spécifiques à chacun des organismes recherchés. Les organismes recherchés amplifiés s'hybrideront chacun avec les sondes qui leur sont spécifiques.

La préparation de sondes différentes permet en outre de réaliser des témoins d'amplification. Ces témoins positifs permettent de valider le résultat. Un ADN témoin ne présentant aucune interaction avec les ADN recherchés est placé dans l'échantillon. On prépare sur le support une sonde spécifique à cet ADN témoin. Si l'amplification et l'hybridation se sont accomplies correctement, l'ADN témoin a été amplifié et hybridé avec sa sonde spécifique. La sonde témoin révélera alors une tâche colorée.

Selon un mode préférentiel la ou lesdites sondes spécifiques sont obtenues par amplification d'un ou plusieurs fragments d'ADN dudit organisme à détecter, notamment par une polymérisation en chaîne (PCR). Ceci permet d'accroître davantage la sensibilité de la méthode.

Cette méthode utilise les avantages d'une réaction d'hybridation réalisée sur support microporeux, inerte vis à vis des réactions chimiques et enzymatiques mises en oeuvre pour révéler le produit recherché, sans pour autant utiliser un appareil pour lire le résultat de l'analyse. Selon un mode préférentiel, le support microporeux est placé au fond de l'un des puits d'une plaque comportant 24 puits. Ainsi, lorsque la plaque est déposée sur un agitateur rotatif, ledit support est en rotation, au fond dudit puit, dans le liquide de lavage. Cette rotation assure un brassage dudit support qui, associé à la température des solutions de lavage, permet d'accroître la contrainte des lavages.

Un autre avantage de la méthode selon l'invention est qu'elle comporte une moyen de contrôle de la réaction d'amplification, utilisant un fragment d'ADN et des amorces de polymérisation choisies pour n'avoir aucune capacité d'hybridation avec l'ADN de l'organisme recherché ou avec les amorces utilisées pour amplifier ledit ADN.

Le marquage peut être réalisé par incorporation d'un adduct à l'ADN amplifié capable de lier une enzyme qui catalyse la formation du produit coloré visible à l'oeil nu.

La méthode comporte également une étape de dilution sérielle du produit recherché et/ou des sondes d'ADN utilisées pour l'identifier, permettant d'évaluer la quantité de produit recherché et de déterminer le seuil de sensibilité de la méthode rapide de détection telle que précédemment décrite.

Un autre objet décrit est un kit permettant de mettre en oeuvre le procédé de détection selon l'invention.

Selon un mode de réalisation, la méthode selon l'invention est spécialement utilisée pour détecter la présence de microorganismes pathogènes et notamment de virus, dans des populations de crustacés.

Les buts, objets et caractéristiques ressortiront mieux à la lecture de la description qui suit faite en référence aux dessins joints dans lesquels :

La figure 1A représente les résultats des tests concernant les témoins internes positifs de PCR, avec dépôt d'un échantillon à trois concentrations différentes.

La figure 1B représente les résultats des tests concernant les témoins internes positifs de PCR, avec différents échantillons.

La figure 2 représente les résultats des tests de détection du virus WSSV, en fonction de différentes conditions d'hybridation.

La figure 3 représente les résultats des tests de sensibilité de la méthode selon l'invention en fonction de la concentration de virions WSSV et le nombre de cycles de PCR.

La figure 4A représente les résultats des tests de détection du virus WSSV en fonction de la variation de la quantité de sondes et de la quantité de cibles.

La figure 4B correspond au graphique représentant l'intensité des taches obtenues lors des tests représentés à la figure 4A.

La figure 5 représente les résultats de la quantification de charge virale par la méthode selon l'invention.

La figure 6 représente les résultats obtenus lors de l'évaluation du seuil de détection de la méthode selon l'invention et la comparaison avec la méthode Dot Blot et la méthode PCR.

La figure 7 représente les résultats de l'analyse par la méthode selon l'invention, de trois tissus dont le tropisme pour le virus WSSV est le plus élevé.

Les exemples suivant explicitent des modes de mise en oeuvre de la présente invention.

**Exemple 1: Préparation de la sonde du virus WSSV (White Spot Syndrome Virus) :**

La souche de White Spot Syndrome Virus (WSSV) utilisée provient de la crevette *Panaeus monodon* et a été fournie par l'Aquaculture Pathology Group (Arizona, USA).

La sonde spécifique du virus WSSV est obtenue par amplification génique, grâce à la technique de polymérisation en chaîne (PCR). Les amorces oligonucléotidiques spécifiques sont choisies pour amplifier un fragment de 500 paires de bases (pb) du clone plasmidique L46. Ce clone, issu d'une banque génomique, contient un fragment du génome du virus WSSV (2200pb) provenant de la crevette *Penaeus chinensis* qui a été montré par hybridation *in situ* et par *Dot Blot* comme étant une séquence signature spécifique du virus WSS (Shi *et al.*, 1998).

Le clone L46 est mis en culture à 37°C toute une nuit sous agitation dans 3 millilitres (ml) de milieu LB liquide contenant 300 microgrammes ($\mu$g) d'ampicilline. L'extraction de l'ADN plasmidique avec l'insert se fait par minipréparation.

L'ADN plasmidique sert ensuite de matrice pour la synthèse de la sonde L46. Le principe consiste à utiliser des amorces spécifiques de l'ADN viral (insert) afin d'obtenir par la technique PCR, un fragment spécifique du génome viral. Dans la solution de réaction sont mélangés 0,5 micromolaires ($\mu$M) de chacune des deux amorces (sL46 ; 3'-CCAAGTACGTAGTGGGTG-5' et asL46 ; 3'-GTATTGCTCACCTCTTCC-5'), 2,5 mM de MgCl$_2$, du tampon d'enzyme (TE) 1X, 4 unités de Taq polymérase, 100 $\mu$M de dATP, dCTP, dGTP, dTTP et 1 $\mu$l de l'ADN plasmidique dilué au 1/20$^e$ (3-4 nanogrammes (ng)). Le volume final est ajusté à 100 $\mu$l avec de l'eau stérile.

L'amplification est réalisée dans les conditions suivantes :

- premier cycle une fois : 2 min à 95°C ;
- deuxième cycle 40 fois : 30 sec à 95°C, 30 sec à 58°C, 1 min à 72°C ;
- troisième cycle une fois : 2 min à 72°C puis à 4°C sans limitation de temps.

Le produit de PCR est purifié sur colonne afin d'enlever les amorces, précipité avec 2,5 volume d'éthanol 100% froid et 0,1 volume d'acétate de sodium 3 M, pH 5, congelé 30 min à 80°C puis centrifugé à 10000 g pendant 30 min. Le surnageant est éliminé et le culot repris dans 500 $\mu$l d'éthanol 70% froid. Après une centrifugation de 10 min à 10000 g, le culot est séché et resuspendu 10 min à 65°C dans du TE 1X, pH 8.

Le produit de PCR purifié et concentré est vérifié sur gel d'agarose 1 % et, après mesure de la densité optique au spectrophotomètre, la sonde est ramenée à la concentration voulue par dilution dans du TE 1X, pH 8. Par approximation, 1 ng d'ADN double brin (db) mesuré au spectrophotomètre correspond à $4.10^9$ molécules d'ADN simple brin (sb).

**Exemple 2: Préparation de la sonde spécifique au témoin interne de PCR**

La sonde correspondant au témoin positif est obtenue par PCR sur un plasmide issu d'une banque SAGE. Ces plasmides contiennent des concatémères formés d'un grand nombre de fragments d'ADN agencés au hasard. Ce caractère aléatoire réduit de manière radicale les risques d'interférence avec le produit recherché. En outre, étant donné qu'une banque de plasmides SAGE contient plusieurs dizaines de milliers de telles séquences aléatoires, il est possible d'en sélectionner une qui ne réagira pas avec le produit recherché, quel qu'il soit. Avec les amorces oligonucléotidiques M13as et M13s, spécifiques du plasmide, un fragment de l'insert de 300 à 500 pb est amplifié dans les mêmes conditions PCR que celles décrites à l'exemple 1. Sans risque de croisement avec l'ADN du microorganisme recherché, ce type de sonde est donc idéal pour constituer un témoin positif.

Ce plasmide SAGE correspondant au témoin positif de PCR est nécessaire pour "valider" le bon déroulement de la PCR et de l'hybridation. Il évite le risque de faux négatifs dus à une inhibition de la PCR causée par l'échantillon à tester ou encore des erreurs de manipulation lors de l'étape de PCR ou d'hybridation. En cas d'inhibition ou d'erreur, le plasmide témoin, présent dans le tube de PCR avec l'échantillon n'est pas amplifié au cours de la PCR multiplexe ou ne s'hybride pas avec sa propre sonde. La validation du résultat d'une membrane n'est donc possible que lorsque la tache colorée du témoin apparaît à l'endroit où est déposée la sonde spécifique au témoin interne. Dans l'invention, il est préférable de déposer à une extrémité de la membrane, des échantillons correspondant au témoin positif de PCR ($2,5.10^{11}$ molécules d'ADN sb).

A côté de ces dépôts de témoins positifs, on réalise un dépôt de témoin négatif d'hybridation (produit de PCR exogène, issu d'un plasmide autre que les plasmides SAGE et L46) et pour lequel aucun spot ne doit apparaître après la révélation colorimétrique.

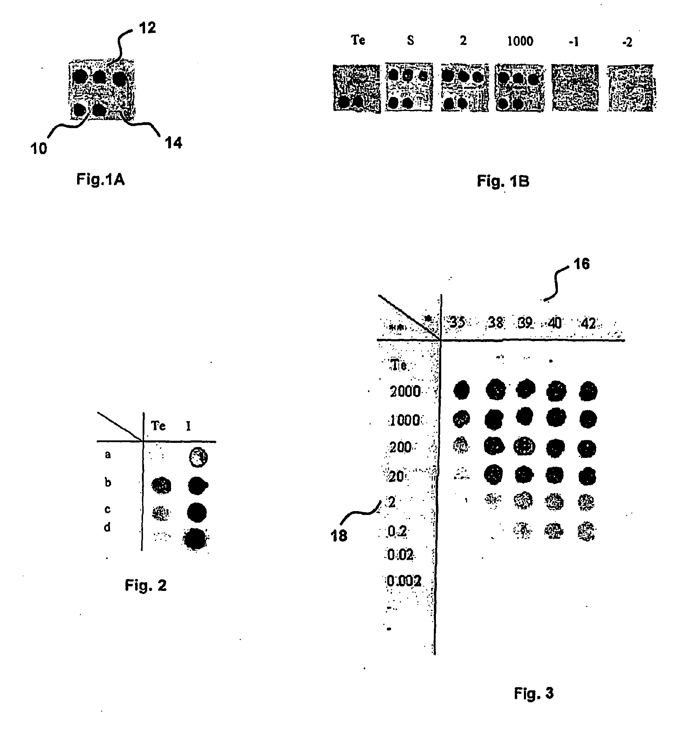

Les résultats sont représentés à la figure 1A représentant la membrane sur laquelle ont été faits les dépôts. A la figure 1A, 10 représente les deux dépôts de témoin positif de PCR, 12 correspond au dépôt du témoin négatif d'hybridation et 14 correspond aux dépôts des trois concentrations décroissantes de la sonde spécifique du microorganisme pathogène.

La figure 1B représente d'autres résultats de PCR avec différents échantillons, permettant de voir l'intérêt des témoins interne PCR, dans laquelle Te correspond au résultat de PCR avec un témoin négatif (hémolymphe non infectée); S, 2 et 1000 correspondent aux résultats de PCR avec des concentrations de cible respectivement égales au seuil de détection, soit $0,2.10^3$ virions/$\mu$l, à $2.10^3$ virions/$\mu$l et $1000.10^3$ virions/$\mu$l d'hémolymphe; -1 correspond à un résultat faux négatif, dont l'absence de témoins PCR est due à une erreur de manipulation (absence de Taq polymérase) et -2 correspond à un résultat faux négatif, dont l'absence de témoins PCR est due à une mauvaise clarification (pas de centrifugation après l'étape de chauffage). Les témoins positifs PCR permettent donc d'être certain que l'absence de taches au niveau des dépôts de cibles n'est pas due à une mauvaise PCR.

**Exemple 3 : Immobilisation de la sonde sur membrane de Nylon**

La sonde est dénaturée 3 min à 95°C et fixée immédiatement dans la glace avant de déposer des échantillons de 1 $\mu$l sur 1 $cm^2$ de membrane de Nylon chargée positivement. Dans le cas de membranes placées dans une plaque de 24 puits, la densité peut aller jusqu'à 9 dépôts par membrane avec un espacement entre les dépôts de 2 mm. Après 10 min de séchage à température ambiante la membrane est préhybridée pendant 30 min, toujours à température ambiante, dans du tampon d'hybridation (6X SSC, 0,1 % N-lauroylsarcosine, 0,02 % SDS, 1 % lait écrémé, 1 % tween 20) puis séchée à l'air libre pour être déposée au fond d'un puit de la plaque à 24 puits. Les membranes sont ainsi conservées et stables à l'abri de la lumière à 4°C.

Les sondes correspondant aux témoins positif et négatif de PCR sont déposée, selon le même protocole que ci-dessus, sur la membrane de Nylon, à côté de la sonde spécifique de l'ADN du WSSV.

- Résultat de l'hybridation de la sonde :

Les résultats de l'hybridation de la sonde sont représentés à la figure 2, dans laquelle Te représente l'échantillon sain (témoin négatif), I l'échantillon infecté, (a), (b), (c) et (d) les différentes conditions d'hybridations :

|  | Sonde | Cible |
|---|---|---|
| **(a)** | Par PCR à partir de 20 ng de matrice plasmidique | Volume d'hybridation de 0,5 ml |
| **(b)** | 80 ng de plasmide L46 | Dépôt direct sur la membrane |
| **(c)** | Par PCR à partir de 20 ng de matrice plasmidique | Dépôt direct sur la membrane |

(suite)

|  | | Sonde | Cible |
|---|---|---|---|
| **(d)** | | Par PCR à partir de 2 ng de matrice plasmidique | Dépôt direct sur la membrane |

Lors la condition (a), le dépôt est effectué directement sur la membrane, ce qui a pour effet de favoriser l'interaction moléculaire entre la sonde et la cible et donc d'augmenter la sensibilité et la rapidité de la méthode.

Avec la condition (b), on peut voir que la fixation sur la membrane du plasmide L46 et non d'un produit de PCR issu du même plasmide augmente de façon importante le bruit de fond par des hybridations non spécifiques. De plus, en comparant les conditions (c) et (d), on peut voir que, lors de l'étape de fabrication de la sonde par amplification génique, la quantité de matrice plasmidique dans le mélange réactionnel de PCR doit être la plus faible possible pour réduire le bruit de fond au maximum.

Pour réduire le bruit de fond, la préparation de la sonde est une étape tout aussi critique que celle du lavage. Une fois ces deux étapes optimisées, le bruit de fond est quasi inexistant. Cependant, il peut arriver que ce dernier apparaisse plus élevé, en particulier si l'étape du « brassage » de la membrane ne s'est pas faite correctement.

**Exemple 4 : Préparation de la cible**

- Clarification à partir de tissus de crustacés dont la contamination est recherchée :

Les pléopodes ou les branchies des crustacés, ici les crevettes Panaeus monodon, sont broyés dans du tampon TN 1X (20 mM Tris-HCl, 0,4 M NaCl, pH 7,4) à raison de 0,1 g de tissus pour 1 g de tampon (dilution au 1/10e). De force ionique élevée, ce tampon est isotonique au milieu intérieur des crustacés marins et limite donc les chocs osmotiques. L'homogénat est centrifugé à 10000 g pendant 10 min afin de précipiter les débris de tissus. Le surnageant est ensuite chauffé 10 min à 100 °C, vortexé puis centrifugé à 10000g pendant 10 min. Pour éliminer tous les agglomérats de tissus et donc prévenir une inhibition de la PCR, le surnageant est centrifugé de nouveau 10 min dans les mêmes conditions.

- Clarification à partir de l'hémolymphe de crustacés dont la contamination est recherchée :

L'hémolymphe, prélevée à l'aide d'une seringue, est immédiatement déposée dans la glace pour éviter toute coagulation. Contrairement aux tissus, l'hémolymphe ne nécessite pas de broyage et donc n'a pas besoin d'être diluée dans du tampon mais est directement centrifugée à 10000 g pendant 10 min pour enlever les débris cellulaires. La suite du protocole de clarification est la même que celle utilisée pour les tissus.

- Evaluation du seuil de détection :

Afin de définir le nombre de cycles de PCR nécessaires pour que la réaction de marquage de la cible soit efficace, un seuil de détection a été déterminé en effectuant des dilutions d'une suspension virale pure dans de l'hémolymphe non infectée.

Cette suspension virale a été obtenue par purification du virus à partir de l'hémolymphe infectée provenant d'une étrille *Liocarcinus vernalis.*

Après mesure au spectrophotomètre, la charge virale de la suspension virale est estimée à 2 millions de virions par $\mu$l. La dilution de cette suspension virale pure dans de l'hémolymphe non infectée a permis de définir un plateau d'amplification à 40 cycles de PCR et un seuil de détection de la méthode de 200 virions par $\mu$l d'hémolymphe.

La suspension de virus purifiés observée en microscopie électronique à transmission est composée essentiellement de nucléocapsides, les enveloppes étant dégradées au cours de la purification. Après mesure au spectrophotomètre, la charge virale de cette suspension est estimée à 2 millions de virions par $\mu$l.

La dilution de cette suspension virale pure dans de l'hémolymphe non infectée a permis de définir le nombre de cycles de PCR nécessaires pour que la réaction de marquage de la cible soit efficace. D'autre part, nous avons pu déterminer un seuil de détection. Les résultats sont représentés à la figure 3. 16 correspond au nombre de cycles de PCR, 18 correspond au nombre de virions par $\mu$l d'hémolyse déposés sur la membrane, Te correspondant au témoin négatif (hémolymphe non infectée). Les résultats montrent que le plateau d'amplification est atteint dès 38 cycles de PCR et le seuil de détection de la méthode est de 200 virions par $\mu$l d'hémolymphe.

- Gamme de variation Sonde/ Cible étudiée par la méthode selon l'invention (avec marquage par 40 cycles de PCR) :

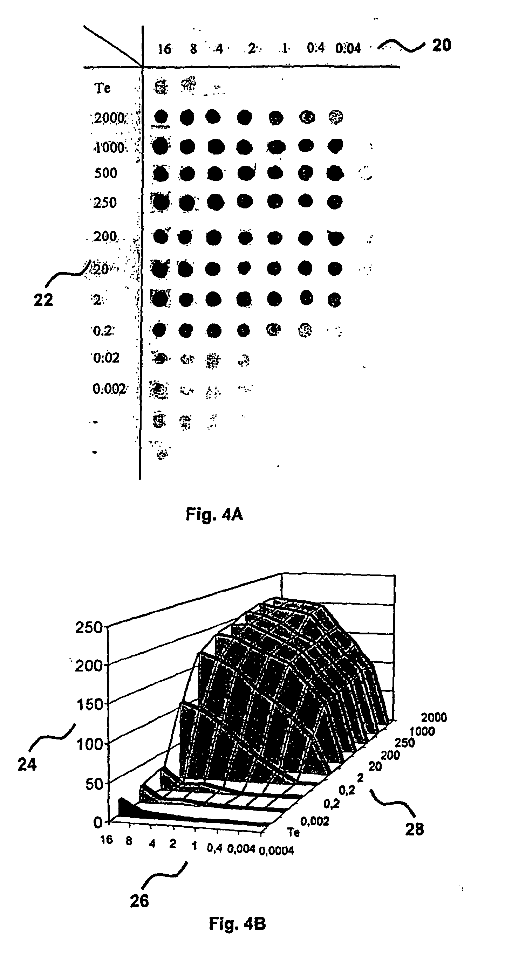

Une gamme de variation sonde/cible a été réalisée. Le nombre de sondes ADN sb déposées sur la membrane varie entre 0,004.$10^{11}$ et 16.$10^{11}$. La quantité de cibles varie entre 0,002.$10^3$ et 2000.$10^3$ virions/$\mu$l d'hémolymphe. Les résultats obtenus sur la membrane, sont représentés à la figure 4A, dans laquelle 20 représente le nombre de sondes ADN sb (x $10^{11}$), 22 représente le nombre de virions/$\mu$l d'hémolymphe (x$10^3$), Te correspondant au témoin négatif (hémolymphe non infectée).

La figure 4B correspond à un graphique représentant l'intensité des taches obtenues sur la membrane en fonction des quantités de sondes et de cibles utilisées. 24 correspond à l'intensité des taches mesurée par le programme ImageTool, 26 représente le nombre de sondes ADN sb (x $10^{11}$) et 28 représente le nombre de virions/$\mu$l d'hémolymphe (x$10^3$), Te correspondant au témoin négatif (hémolymphe non infectée).

Les résultats montrent que, lorsqu'un nombre de sondes inférieur à 0.04 $10^{11}$ molécules d'ADN sb (1 ng) est déposé sur la membrane de Nylon, aucune tache n'apparaît, même avec de fortes concentrations en cibles marquées. Au-delà de 4.$10^{11}$ molécules d'ADN sb (100ng) déposées, l'intensité des tache atteint un plateau avec les fortes concentrations en cibles et du bruit de fond apparaît sur les membranes pour les témoins négatifs.

Avec la fraction contenant 2.$10^6$ virions/$\mu$l, une inhibition partielle de la PCR est observée. Cela suggère que la charge virale est trop élevée: l'excès de protéines virales et/ou d'inhibiteurs viraux ralentissent l'amplification mais ne l'inhibent jamais complètement. Cette observation montre à quel point il est important de tester une gamme de dilutions à partir des clarifications.

## Exemple 5 : Amplification PCR de la cible et marquage à la Digoxygénine

Le marquage de l'ADN cible, issu des crustacés à diagnostiquer, est réalisé par amplification génique où une partie des molécules de dTTP est remplacée par le dUTP marqué à la Digoxygénine. L'amplification par PCR du génome viral permet de tester des échantillons faiblement infectés. Dans la solution de réaction sont mélangés 0,5 $\mu$M de chacune des deux amorces (sL46 ; 3'-CCAAGTACGTAGTGGGTG-5' et asL46 ; 3'-GTATTGCTCACCTCTTCC-5'), 0,1 $\mu$M de chacune des deux amorces M13s et M1 3as, 10 picogrammes (pg) du plasmide correspondant au témoin interne de PCR, 2,5 mM de MgCl$_2$, du tampon d'enzyme 1X, 2 unités de Taq polymérase, 200 $\mu$M de dATP, dCTP, dGTP, 190 $\mu$M de dTTP, 10 $\mu$M de digoxygénine-11-dUTP et 1$\mu$l de la clarification à tester. L'amplification est réalisée dans les conditions suivantes :

- premier cycle une fois : 2 min à 95°C;
- deuxième cycle 40 fois : 30 sec à 95°C, 30 sec à 62°C, 1min à 72°C;
- troisième cycle une fois : 2 min à 72°C;
- quatrième cycle une fois: 2min à 95°C puis à 4°C sans limitation de temps.

## Exemple 6 : Hybridation de la cible sur la membrane de Nylon

Les 50 $\mu$l du produit de PCR correspondant à la cible sont déposés sur la membrane pour une hybridation de 30min à température ambiante. Ensuite, le filtre est lavé brièvement à température ambiante dans 1ml de 2X SSC contenant 0,1 % de SDS, puis deux fois 5 min à température ambiante dans 1 ml de cette même solution et enfin trois fois 10 min dans 1ml d'une solution chauffée entre 40 et 50°C, composée de 0,1X SSC contenant 0,1 % de SDS. Afin de décrocher les cibles mésappariées, les lavages se font sous agitation continue et forte stringence.

## Exemple 7 : Révélation colorimétrique et analyse d'image

Après l'hybridation, la membrane est bloquée à température ambiante pendant 15 min dans 0,5 ml d'une solution TBS 1X (10 mM Tris HCl, pH 7,4, 150 mM NaCl, 0,3 % BSA), 1 % lait écrémé, 2 % sulfate de dextran. La membrane est ensuite rincée brièvement au TBS 1X.

L'anticorps anti-digoxigénine conjugué à la phosphatase alcaline, est employé pour détecter les spots sur la membrane. Pour cela, le filtre est incubé 30 min à température ambiante avec 0,5 ml du tampon TBS 1X contenant une dilution au 1/5000 de l'anticorps (soit une activité enzymatique de 150 mU/ml). La membrane est ensuite lavée sous agitation à température ambiante quatre fois 10 min dans 1 ml de TBS 1X puis équilibrée 2 min dans 0,5 ml d'une solution composée de 100 mM Tris HCl, pH 9,5 (20°C) , 100 mM NaCl, 50 mM MgCl$_2$. Puis, le filtre est' incubé avec 0,5 ml de cette même solution contenant 100 $\mu$g de NitroBlueTetrazolium (NBT) et 100 $\mu$g de 5 Bromo-4 Chloro-3 Indolyl Phosphate (BCIP).

Après 10 min de révélation, le développement colorimétrique est stoppé en incubant la membrane 5 min dans du PBS 1X contenant 20 mM EDTA. La membrane est conservée dans cette solution à l'abri de la lumière.

L'image peut ensuite être numérisée grâce à un scanner, puis l'intensité des taches est mesurée par un logiciel calculant le niveau de gris moyen par tache. Les valeurs mesurées sont ensuite inversées pour avoir des intensités comprises entre 0 (blanc) et 255 (noir).

L'image peut également être facilement analysée à l'oeil nu.

**Résultats :**

Quantification de l'infection:

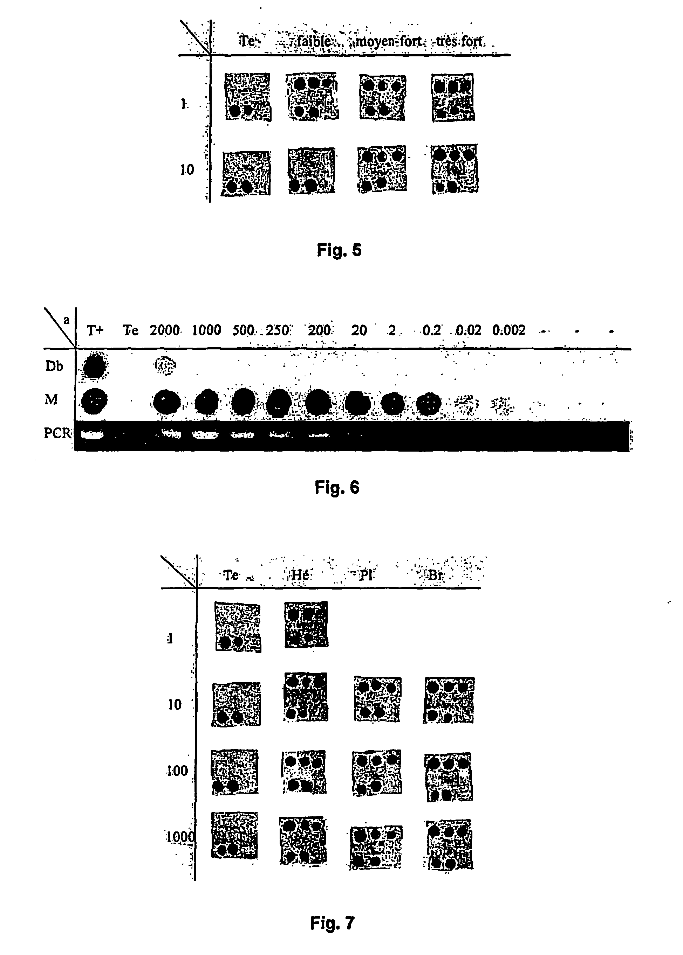

Les résultats de la quantification de la charge virale par la méthode selon l'invention sont représentés à la figure 5, dans laquelle Te, faible, moyen-fort et très fort représentent les niveaux d'infections, Te correspondant à l'échantillon non-infecté, et 1 et 10, les dilutions dans l'eau stérile de l'échantillon analysé (hémolymphe), 1 correspondant à l'échantillon non dilué et 10 à l'échantillon dilué au 1/10ème. L'analyse de l'intensité des taches sur deux membranes par animal contaminé à différents stades de l'infection permet d'appréhender le niveau de l'infection. Pour un animal sain (Te), aucune tache spécifique de l'organisme pathogène ne doit apparaître sur les deux membranes. Pour un animal faiblement infecté, les taches de faible intensité se révèlent uniquement sur la première membrane. Pour les échantillons moyennement à fortement infectés, l'intensité des taches est élevée sur les deux membranes. Enfin, si un animal est fortement infecté, il y aura inhibition partielle de la PCR avec la fraction non diluée de l'hémolymphe et l'intensité des taches est élevée uniquement sur la deuxième membrane.

- Les contrôles :

Pour valider un résultat positif à la détection du WSSV, l'agent pathogène, dans un échantillon (apparition d'un spot coloré), il est important de réserver sur la plaque, deux membranes pour le seuil de détection préalablement déterminé (200 virions/µl). Ce seuil peut être obtenu avec un mélange de PCR contenant le plasmide L46 calibré et donnant l'équivalant du seuil de détection. Pour qu'un échantillon soit WSSV positif, les membranes devront avoir une intensité des taches proche ou supérieure au seuil. Ainsi, les risques de faux positifs dus à un bruit de fond trop élevé (mauvais lavages) sont totalement écartés.

Afin d'effectuer les contrôles négatifs, deux membranes sont hybridées à partir d'un mélange de PCR contenant uniquement le plasmide "témoin interne de PCR". L'analyse d'un échantillon se fait sur deux membranes: la première est testée avec la fraction non diluée de l'échantillon de tissu de l'animal testé et la seconde avec la fraction diluée au 1/10ème. Avec deux membranes pour témoins négatifs et deux autres pour le seuil de détection, 20 membranes soit 10 échantillons par plaque de 24 puits peuvent être analysés.

**Exemple 8 : Comparaison des sensibilités des méthodes *Dot Blot et la méthode de détection* selon l'invention**

- Le Dot blot :

Dans la méthode *Dot Blot,* la "cible" correspond à l'échantillon d'ADN à tester fixé sur la membrane et la "sonde" fait référence aux molécules d'ADN marquées et présentes dans la solution d'hybridation.

- Préparation de la cible

Les clarifications des échantillons sont obtenues selon les mêmes protocoles développés dans la méthode selon l'invention. Après dénaturation 3 min à 95°C et fixation dans la glace, des échantillons de 1 µl de ces clarifications sont déposés sur la membrane de Nylon. Après 10 min de séchage à température ambiante, la membrane est préhybridée 1 h à 68°C dans 2 ml de tampon d'hybridation (5X SSC, 0,1% N-lauroylsarcosine, 0,02 % SDS, 0,5 % Bloking reagent, Roche Diagnostic).

- Préparation de la sonde et hybridation sur la membrane de Nylon

L'ADN plasmidique du clone L46 sert de matrice pour la synthèse de la sonde par PCR. Le protocole est le même que celui utilisé dans la méthode selon l'invention, à ceci près que la sonde est marquée en incorporant dans la solution de réaction 200 µM de dATP, dCTP, dGTP, 190µM de dTTP, 10 µM de digoxygénine-11-dUTP.

La sonde est ajoutée dans la solution de préhybridation à la concentration finale de 800 ng/µl pour une incubation d'une nuit à 68°C.

Le lendemain, quatre lavages à TA sont effectués pour éliminer les sondes non hybridées; deux lavages de 5 min dans 50 ml de tampon 2X SSC contenant 0,1 % de SDS puis deux lavages de 15 min dans 50 ml de tampon 0,1X SSC contenant 0,1 % de SDS.

- Révélation colorimétrique et analyse d'image

Après l'hybridation, la membrane est bloquée à TA pendant 30 min 0dans 50 ml d'une solution TBS 1X, 0,5 % Bloking reagent. Le filtre est ensuite incubé 30 min à température ambiante dans 2 ml de tampon TBS 1X contenant une dilution au 1/5000 de l'anticorps. Le filtre est ensuite lavé deux fois 15 min avec 50 ml de tampon TBS 1X puis incubé dans le noir avec 2 ml de la solution de révélation contenant 200 $\mu$g de NBT et 200 $\mu$g de BCIP. Le développement colorimétrique est stoppé après 2 h de révélation par incubation dans une solution PBS 1X, 20 mM EDTA.

Le plasmide, qui possède la séquence L46, est déposé sur la membrane et sert de témoin positif. Un témoin négatif, constitué d'hémolymphe non infectée, est également déposé sur la membrane.

**Comparaison de la sensibilité de la méthode selon l'invention et du *Dot Blot***

- Purification du virus à partir de l'hémolymphe :

L'hémolymphe infectée (6 ml) provenant de *Liocarcinus vernalis* (étrille) est broyée dans du tampon TN 1X (0,02 M Tris-HCl, 0,4 M NaCl, pH 7,4) puis clarifiée à 1400 g pendant 10 min pour enlever les débris cellulaires. Le surnageant est centrifugé à 113000 g pendant 1 h (L7 Beckman). Puis, le culot de cette deuxième centrifugation est remis en suspension dans 1 ml de tampon TN 1X et déposé sur un gradient continu, préformé de sucrose 20-50 % préparé à l'aide d'un générateur de gradient (Autodensiflow, Büchler). Après une centrifugation d'une heure à 150000 g, la bande virale est collectée à l'aide d'un fractionneur de gradient (Gilson, Fc203) et analysée en continu à la longueur d'onde de 254 nm avec un spectrophotomètre UV (ISCO UA6). Pour éliminer le sucrose, la fraction contenant les virus est diluée dans du tampon TN 1X et centrifugée à 113000 g pendant 1h. Le culot final est repris dans 500 $\mu$l de tampon TN 1X.

La suspension de virus purifiée est ensuite déposée sur des grilles collodionnées-carbonées et contrastées négativement avec l'acide phosphotungstique (APT) à 2 %, pH 7 et la qualité de la purification est contrôlée en microscopie électronique à transmission (Hitachi HU11C).

Le même protocole est réalisé sur de l'hémolymphe saine, prélevée chez un crabe de la même espèce. Cette hémolymphe contrôlée en microscopie électronique sert de blanc pour la quantification de la charge virale.

- Extraction de l'acide nucléique viral :

La suspension de virus purifiée (300 $\mu$l des 500 $\mu$l) est traitée à la protéinase K (200 $\mu$g/ml) en incubant 2 h à 37°C, puis au SDS (0,5 %) en incubant 1 h à 50°C. La déprotéinisation se fait ensuite par une extraction avec 1 volume de Phénol/Chloroforme/Alcool isoamylique (25:24:1). Après centrifugation à 10000 g pendant 5 min, l'acide nucléique contenu dans la phase aqueuse est précipité avec 2,5 volumes d'éthanol 100 % froid et 1 volume composé au 9/10$^{\text{ème}}$ de la phase aqueuse et au 1/10$^{\text{ème}}$ d'acétate de sodium, 3 M. Après 30 min à -80°C et une centrifugation à 10000g de 30 min, le culot est lavé à l'éthanol 70 % froid, séché à 37°C puis resuspendu 10 min à 65°C dans 100 $\mu$l de TE 1X, pH 8.

- Quantification de la charge virale

La suspension d'ADN est analysée dans le proche UV (220-320 nm) à l'aide d'un spectrophotomètre enregistreur à double faisceaux (Safas 190 DES). La lecture de l'Absorbance à 260 nm ($A_{260}$) permet de déterminer la concentration de l'ADN présent dans l'échantillon par l'équation suivante:
pour de l'ADNdb :

$$C\ (\mu g/ml)\ =\ A_{260}/0,02$$

Le bruit de fond est enlevé par soustraction de l'$A_{260}$ sur de l'hémolymphe saine.

Un virion contient une seule molécule d'ADNdb circulaire, dont la taille est de 305000 pb (Xu, 2000). En fixant 327 daltons (Da) par XMP de l'acide nucléique 1 ng d'ADNdb/$\mu$l, mesuré au spectrophotomètre, est estimé correspondre à 3 millions de virions/$\mu$l de la suspension virale pure.

Une fois la charge virale calculée, le reste de la suspension de virus purifiés est ensuite diluée de façon logarithmique dans de l'hémolymphe non infectée, contrôlée en microscopie électronique. La sensibilité de la méthode de détection selon l'invention est alors comparée à celle du Dot Blot selon les protocoles vus précédemment.

**Résultats :**

Les résultats sont représentés à la figure 6. Sur cette figure, on peut voir les résultats obtenus avec la méthode de détection selon l'invention (M), la méthode Dot Blot (Db) et la technique PCR. Pour cette dernière, les résultats sont observés après 40 cycles et migration par électrophorèse sur gel d'agarose. Les résultats de la méthode selon l'invention sont obtenus pour un nombre de sondes égal à $4.10^{11}$ molécules d'ADN sb et 40 cycles de PCR pour le marquage. 30 correspond au nombre de virions/$\mu$l d'hémolymphe ($x10^3$), T+ correspondant au témoin positif (plasmide L46 pour les méthodes Dot Blot et PCR et témoin interne SAGE pour la méthode selon l'invention avec un nombre de sondes égal à $2,5.10^{11}$ molécules sb). Au vu de ces résultats, on constate que la sensibilité de la méthode selon l'invention) est quatre ordres de grandeur (4 logs) supérieure à celle du Dot Blot et deux ordres de grandeur (2 logs) supérieure à l'observation d'un produit de PCR après migration sur gel d'électrophorèse. Ces résultats, vérifiés à deux reprises font de la méthode selon l'invention, une technique très efficace pour la détection du WSSV.

- Autres facteurs favorisant la sensibilité :

La séquence cible à amplifier contient 65 % de A-T, ce qui favorise l'incorporation du dUTP marqué à la Digoxygénine lors de la préparation de la cible. L'hybridation du produit de PCR marqué se fait sur un autre produit d'amplification, ce qui favorise la fixation d'un maximum de molécules cibles sur les sondes. Un grand nombre de molécules d'anticorps peut alors se fixer à la Digoxygénine. Il en résulte que l'action catalytique de la phosphatase alkaline est favorisée, la sensibilité de la méthode est alors très élevée et le temps de révélation très court.
Les premières taches apparaissent quelques secondes après le début de l'étape de révélation. Le temps de développement colorimétrique a été fixé à 10 min afin de pouvoir comparer toutes les membranes lors des différents tests. Ce temps permet d'obtenir une gamme d'intensité des taches en corrélation avec le degré de l'infection, au-delà, un bruit de fond trop élevé apparaît, pour un échantillon sain. Le développement est stoppé en lavant la membrane dans une solution contenant de l'EDTA.

**Exemple 10 : Choix du tissu à analyser lors d'un diagnostic de contamination virale chez des crustacés**

La détection du WSSV a été étudiée avec la méthode selon l'invention à partir des trois principaux tissus dont le tropisme (c'est à dire là où le virus se développe le mieux) pour le WSSV est le plus élevé, soit l'hémolymphe, les branchies et les pléopodes (Lo et al., 1997). Ces différents tissus ont été prélevés sur un même animal (cigale de mer, *Scyllarus arctus*) infecté et mort suite au WSS (White Spot Syndrome).
Les protocoles de clarification à partir de ces trois tissus sont décrits dans l'exemple 4.

**Résultats :**

La figure 7 montre les résultats d'analyse, par la méthode selon l'invention, sur les trois tissus décrits ci-dessus. Les références Te, Hé, Pl et Br correspondent respectivement au témoin négatif, c'est à dire de l'hémolymphe non infectée prélevée avant l'inoculation du virus, à l'hémolymphe infectée, aux pléopodes infectés et aux branchies infectées. Les références 1, 10, 100 et 1000 correspondent au facteur de dilution de l'échantillon testé, soit respectivement non dilué, dilué au 1/10ème, dilué au 1/100ème, et dilué au 1/1000ème.
Conformément à l'explication de la présentation des résultats de la figure 1B, on constate que le virus WSSV peut être détecté, par la méthode selon l'invention, dans les trois tissus analysés.
L'hémolymphe est le tissu possédant le tropisme le plus élevé pour le virus (Lo et al., 1997). Le WSSV y est essentiellement présent sous forme extracellulaire, ce qui évite une étape de broyage du tissu prélevé chez l'animal, rendant ainsi le protocole plus simple et prévenant les contaminations inter-échantillons.
De plus, chez les animaux adultes présents dans les bassins, l'hémolymphe peut être analysée sans dilution ce qui permet de tester des animaux faiblement infectés. Les branchies et les pléopodes nécessitant un broyage dans du tampon de clarification, il n'est possible de les tester que dilués au 1/10ème. Ce problème n'est pas rencontré avec l'hémolymphe, ce qui fait de ce tissu une source idéale pour tester des animaux faiblement infectés ou en latence virale.
La fraction diluée au 1/10ème présente des taches plus intenses que la fraction non diluée de l'hémolymphe. Cette observation montre l'importance d'effectuer une gamme de dilutions à partir de la clarification.
Enfin, l'analyse de l'hémolymphe d'un animal permet d'écarter tout risque de faux positifs dus à une contamination d'un autre animal. En effet, l'hémolymphe est le « sang » du crustacé et le virus se trouve donc dans le milieu intérieur du crustacé. D'autres tissus, tels que les branchies ou les pléopodes, peuvent être « porteurs » de virus provenant d'une contamination de l'eau du bassin sans que l'animal lui-même soit infecté.
Le prélèvement de l'hémolymphe est effectué via une seringue afin de ne pas sacrifier l'animal testé. Ainsi, une des applications de la méthode pourrait être dans la sélection des animaux géniteurs sains ou résistants (permettrait d'éliminer systématiquement les animaux porteurs du virus).

Pour éviter sa coagulation, l'hémolymphe prélevée est simplement placée dans un bac à glace (pas de traitement par un anticoagulant risquant d'inhiber la PCR de marquage).

De plus, il n'est pas possible de mettre plus d'1 µl de la clarification issue d'un broyage (des branchies ou des pléopodes) dans les 50 µl de mélange réactionnel de PCR sans inhiber totalement la réaction de polymérisation. En effet, il semble que, suite au broyage de ces tissus, un excès d'inhibiteurs soit dégagé.

La présente invention comprend également l'utilisation d'un kit pour la mise en oeuvre de la méthode de détection de microorganismes. Ce kit comprend :

- une plaque 24 puits contenant les membranes prêtes à l'emploi,
- un mélange de PCR,
- une solution seuil pour la PCR,
- des solutions pour l'hybridation, les lavages et la révélation colorimétrique.

Le mélange de PCR utilisé dans le kit selon l'invention possède la composition suivante:

eau 313.2µl
Tampon 10x 135µl (Proméga)                                                                                              (1x)
MgCl₂ 135µl (Proméga)                                                                                          (2.5mM)
DNTP - digoxygénine 135µl (Roche Diagnostic): dATP, dCTP, dGTP, 190µM dTTP, 10µM dUTP-Dig)     (200µM
Amorce sens WSSV 135µl                                                                                        (0.5µM)
Amorce anti-sens WSSV 135µl                                                                                  (0.5 µM)
Amorce sens Témoin interne 27µl                                                                              (0.1µM)
Amorce anti-sens Témoin interne 27µl                                                                         (0.1µM)
Plasmide Témoin interne 27µl                                                                                   (10pg)

La solution seuil utilisé dans le kit comprend de l'eau stérile et le plasmide WSSV calibré.

Le kit comprend également 6 solutions tampons utilisés pour l'hybridation, les lavages et la révélation colorimétrique. :

- Solution 1 (78ml) :
  2X SSC, 0.1% SDS
- Solution 2 (78ml) :
  0.1X SSC, 0.1% SDS
- Solution 3 (13ml) :
  TBS 1X (10mM Tris HCl, pH 7.4 (4°C), 150mM NaCl, 0.3% BSA)
  1% Lait écrémé
  2% Sulfate de Dextran
- Solution 4 (117ml) :
  TBS 1X
- Solution 5 (26ml) :
  100 mM Tris HCl, pH 9.5 (20°C), 100mM NaCl, 50mM MgCl₂
- Solution 6 (26ml) :

  PBS 1X
  20mM EDTA
- Solution de révélation (dans un tube de 15ml):
  2.6mg de NitroBlueTetrazolium (NBT)
  2.6mg de 5 Bromo-4 Chloro-3 Indolyl Phosphate (BCIP).

L'utilisateur du kit doit se procurer, avant toute utilisation, la taq polymérase à 5 unités/µl ainsi que les anticorps anti-digoxigénine (fragment Fab), bien connus de l'homme du métier et classiquement utilisés dans les laboratoires. Il doit également posséder l'appareillage nécessaire soit une centrifugeuse, une plaque chauffante, un thermocycleur pour les réactions de PCR et un agitateur rotatif.

Toutefois, selon un autre mode de réalisation, le kit comprend également une solution contenant la taq polymérase à 5unités/µl et une solution d'anticorps anti-digoxigénine (fragment Fab).

**Revendications**

1.  Méthode rapide de détection des ADN d'un ou de plusieurs organismes différents dans un échantillon, **caractérisé en ce qu'**elle consiste à :

    - préparer au moins une sonde spécifique dudit ou desdits organismes,
    - immobiliser ladite sonde sur un support approprié,
    - saturer le support avec des fragments d'ADN ne réagissant pas avec les produits recherchés pour éviter toute interaction non spécifique desdits produits avec le support lui-même,
    - extraire l'ADN dudit ou desdits organismes contenus dans l'échantillon provenant d'un produit ou un sujet,
    - amplifier ledit ou lesdits ADN du ou des organismes simultanément, afin d'obtenir un ou plusieurs amplicons,
    - marquer lesdits amplicons,
    - hybrider à température ambiante par dépôt direct l'ADN amplifié et marqué avec la sonde immobilisée sur le support,
    - révéler le marquage et,
    - examiner directement le résultat, ou obtenir une image numérique de la membrane colorée.

2.  Méthode de détection selon la revendication 1, dans laquelle l'amplification des ADN et le marquage desdits amplicons sont effectués simultanément.

3.  Méthode de détection selon l'une des revendications précédentes, dans laquelle l'amplification est une polymérisation en chaîne (PCR).

4.  Méthode de détection selon l'une des revendications précédentes, comprenant également une étape de préparation d'une sonde témoin d'amplification ne présentant aucune interaction avec les autres fragments d'ADN recherchés.

5.  Méthode de détection selon l'une des revendications précédentes, dans laquelle la ou lesdites sondes spécifiques sont obtenues par amplification d'un ou plusieurs fragments d'ADN dudit organisme à détecter, notamment par une polymérisation en chaîne (PCR).

6.  Méthode de détection selon l'une des revendications précédentes, dans laquelle ledit support est un support microporeux doté de capacité d'adsorption de l'ADN.

7.  Méthode de détection selon la revendication 6, dans laquelle ledit support est inerte vis à vis des réactions chimiques et enzymatiques mises en oeuvre pour révéler le produit recherché.

8.  Méthode de détection selon la revendication 7, dans laquelle ledit support est placé au fond de l'un des puits d'une plaque de 24 puits.

9.  Méthode de détection selon la revendication 7 ou 8, dans laquelle ledit support est une membrane de nylon.

10. Méthode de détection selon l'une des revendications précédentes, dans laquelle ledit marquage consiste à obtenir un produit coloré stable détectable par examen visuel direct et/ou grâce à un dispositif d'analyse d'image.

11. Méthode de détection selon la revendication 10, dans laquelle ledit marquage est un marquage par un produit d'addition incorporé à l'ADN amplifié capable de lier une enzyme qui catalyse la formation du produit coloré visible à l'oeil nu.

12. Application de la méthode de détection selon l'une des revendications 1 à 11, pour la détermination de la contamination de crustacés par le virus WSSV.

13. Utilisation d'un kit pour la mise en oeuvre de la méthode de détection selon l'une des revendications 1 à 11 **caractérisé en ce que** ledit kit comprend :

    - une plaque multipuits ou tout autre plaque de microcupules contenant le support prêt à l'emploi,
    - un mélange de PCR,
    - une solution seuil pour la PCR,
    - des solutions pour l'hybridation, les lavages et la révélation colorimétrique.

14. Utilisation d'un kit selon la revendication 13, dans lequel mélange de PCR possède la composition suivante :

| | |
|---|---|
| eau 313.2μl | |
| Tampon 10x 135μl | (1x) |
| MgCl$_2$ 135μl | (2.5mM) |
| DNTP - digoxygénine 135μl (: | (200μM dGTP, 190μM dTTP, 10μM dUTP-Dig) dATP, dCTP, |
| Amorce sens WSSV 135μl | (0.5μM) |
| Amorce anti-sens WSSV 135μl | (0.5μM) |
| Amorce sens Témoin interne 27μl | (0.1μM) |
| Amorce anti-sens Témoin interne 27μl | (0.1μM) |
| Plasmide Témoin interne 27μl | (10pg) |

## Claims

1. A fast method for detecting the DNAs of one or more different organisms in a sample, **characterised in that** it consists in:

   - preparing at least one specific probe of said organism or organisms,
   - immobilising said probe on an appropriate substrate,
   - saturating the substrate with DNA fragments which do not react with the products sought in order to avoid any non-specific interaction of said products with the substrate itself,
   - extracting the DNA from said organism or organisms contained in the sample coming from a product or a subject,
   - amplifying simultaneously said DNA or DNAs of the organism or organisms, in order to obtain one or more amplicons,
   - labelling said amplicons,
   - hybridising, at ambient temperature by direct deposition, the amplified and labelled DNA with the probe immobilised on the substrate,
   - revealing the labelling and,
   - directly examining the result, or obtaining a digital image of the coloured membrane.

2. The detection method according to claim 1, wherein the amplification of the DNAs and the labelling of said amplicons are carried out simultaneously.

3. The detection method according to one of the preceding claims, wherein the amplification is a chain polymerisation (PCR).

4. The detection method according to one of the preceding claims, also including a step of preparing an amplification control probe which does not have any interaction with the other DNA fragments sought.

5. The detection method according to one of the preceding claims, wherein said specific probe or probes are obtained by amplification of one or more DNA fragments of said organism to be detected, notably by a chain polymerisation (PCR).

6. The detection method according to one of the preceding claims, wherein said substrate is a microporous substrate provided with the ability to adsorb DNA.

7. The detection method according to claim 6, wherein said substrate is inert with regard to the chemical and enzymatic reactions implemented to reveal the product sought.

8. The detection method according to claim 7, wherein said substrate is placed at the bottom of one of the wells of a 24-well plate.

9. The detection method according to claim 7 or 8, wherein said substrate is a nylon membrane.

10. The detection method according to one of the preceding claims, wherein said labelling consists in obtaining a stable coloured product which can be detected by direct visual examination and/or by means of an image analysis device.

11. The detection method according to claim 10, wherein said labelling is a labelling by an additive incorporated into the amplified DNA capable of binding an enzyme which catalyses the formation of the coloured product which is visible to the naked eye.

12. Application of the detection method according to one of claims 1 to 11, to determine the contamination of crustaceans with the WSSV virus.

13. Use of a kit to implement the detection method according to one of claims 1 to 11 **characterised in that** said kit includes:

- a multi-well plate or any other microwell plate containing the ready-to-use substrate,
- a PCR mixture,
- a threshold solution for the PCR,
- solutions for the hybridisation, the washes and the colorimetric revelation.

14. The use of a kit according to Claim 13, wherein PCR mixture has the following composition:

water 313.2$\mu$l
buffer 10x135$\mu$l (1x)
$MgCl_2$,135$\mu$l (2.5mM)
DNTP - digoxigenin 135$\mu$l (: (200$\mu$M dATP, dCTP, dGTP, 190$\mu$M dTTP, 10$\mu$M dUTP-Dig)
WSSV sense primer 135$\mu$l (0.5$\mu$M)
WSSV antisense primer 135$\mu$l (0.5$\mu$M)
Internal control sense primer 27$\mu$l (0.1$\mu$M)
internal control antisense primer 27$\mu$l (0.1$\mu$M)
Internal control plasmid 27$\mu$l (10pg)

**Patentansprüche**

1. Ein Schnellverfahren zum Nachweis von DNAs eines oder mehrerer unterschiedlicher Organismen in einer Probe, **dadurch gekennzeichnet, dass** es aus Folgendem besteht:

- Herstellen zumindest einer spezifischen Sonde aus dem oder den Organismen,
- Immobilisieren der Sonde auf einem geeigneten Träger,
- Sättigen des Trägers mit DNA-Fragmenten, die nicht mit den gesuchten Produkten reagieren, um jegliche nicht spezifische Interaktion der Produkte mit dem Träger selbst zu verhindern,
- Extrahieren der DNA aus dem oder den Organismen, der/die in der Probe, welche von einem Produkt oder einer Versuchsperson stammt, enthalten ist/sind,
- simultanes Amplifizieren der DNA oder der DNAs des oder der Organismen, um ein oder mehrere Amplikons zu erlangen,
- Markieren der Amplikons,
- Hybridisieren bei Raumtemperatur durch Direktablagerung der amplifizierten und markierten DNA mit der auf dem Träger immobilisierten Sonde,
- Hervorheben der Markierung, und
- direktes Untersuchen des Resultats oder Erlangen eines digitalen Bildes der gefärbten Membran.

2. Verfahren zum Nachweis gemäß Anspruch 1, wobei die Amplifikation der DNAs und die Markierung der Amplikons simultan durchgeführt werden.

3. Verfahren zum Nachweis gemäß einem der vorhergehenden Ansprüche, wobei die Amplifikation eine Polymerase-Kettenreaktion (PCR) ist.

4. Verfahren zum Nachweis gemäß einem der vorhergehenden Ansprüche, ebenfalls umfassend einen Schritt des Herstellens einer Amplifikations-Vergleichssonde, die keinerlei Interaktion mit den anderen gesuchten DNA-Fragmenten aufweist.

5. Verfahren zum Nachweis gemäß einem der vorhergehenden Ansprüche, wobei die spezifische(n) Sonde(n) durch die Amplifikation von einem oder mehreren DNA-Fragmenten des nachzuweisenden Organismus, insbesondere durch eine Polymerase-Kettenreaktion (PCR), erlangt wird/werden.

6. Verfahren zum Nachweis gemäß einem der vorhergehenden Ansprüche, wobei der Träger ein mikroporöser Träger ist, der für die Adsorptionsfähigkeit der DNA dotiert ist.

7. Verfahren zum Nachweis gemäß Anspruch 6, wobei der Träger gegenüber den chemischen und enzymatischen Reaktionen, die durchgeführt werden, um das gesuchte Produkt hervorzuheben, inert ist.

8. Verfahren zum Nachweis gemäß Anspruch 7, wobei der Träger auf den Boden eines Wells von einer 24-Well-Platte gegeben wird.

9. Verfahren zum Nachweis gemäß Anspruch 7 oder 8, wobei der Träger eine Nylonmembran ist.

10. Verfahren zum Nachweis gemäß einem der vorhergehenden Ansprüche, wobei die Markierung darin besteht, ein stabiles gefärbtes Produkt zu erlangen, das durch direkte visuelle Untersuchung und/oder dank einer Bildanalysevorrichtung nachweisbar ist.

11. Verfahren zum Nachweis gemäß Anspruch 10, wobei die Markierung eine Markierung durch ein in die amplifizierte DNA eingebrachtes Additionsprodukt ist, das in der Lage ist, ein Enzym zu binden, das die Bildung des mit bloßem Auge wahrnehmbaren gefärbten Produktes katalysiert.

12. Eine Anwendung des Verfahrens zum Nachweis gemäß einem der Ansprüche 1 bis 11 zur Bestimmung der Ansteckung bei Krustentieren mit dem WSSV-Virus.

13. Eine Verwendung eines Kits zur Durchführung des Verfahrens zum Nachweis gemäß einem der Ansprüche 1 bis 11, **dadurch gekennzeichnet, dass** das Kit Folgendes umfasst:

   - eine Multiwellplatte oder eine beliebige andere Mikrowellplatte, die den gebrauchsfertigen Träger enthält,
   - eine PCR-Mischung,
   - ein Grenzwert-Lösemittel für die PCR,
   - Lösemittel für die Hybridisierung, die Spülungen und die kolorimetrische Entwicklung.

14. Verwendung eines Kits gemäß Anspruch 13, wobei die PCR-Mischung die folgende Zusammensetzung aufweist:

| | |
|---|---|
| Wasser 313,2 $\mu$l | |
| Puffer 10 x 135 $\mu$l | (1 x) |
| MgCl$_2$, 135 $\mu$l | (2,5 mM) |
| dNTP - Digoxygenin 135 $\mu$l (: | (200 $\mu$M dATP, dCTP, dGTP, 190 $\mu$M dTTP, 10 $\mu$M DIG-dUTP) |
| Sense-Primer WSSV 135 $\mu$l | (0,5 $\mu$M) |
| Antisense-Primer WSSV 135 $\mu$l | (0,5 $\mu$M) |
| Sense-Primer interne Vergleichsprobe 27 $\mu$l | (0,1 $\mu$M) |
| Antisense-Primer interne Vergleichsprobe 27 $\mu$l | (0,1 $\mu$M) |
| Plasmid interne Vergleichsprobe 27 $\mu$l | (10 $\mu$g) |

EP 1 330 542 B1

Fig.1A

| Te | S | 2 | 1000 | -1 | -2 |

Fig. 1B

Fig. 2

Fig. 3

**Fig. 4A**

**Fig. 4B**

**Fig. 5**

**Fig. 6**

**Fig. 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **M. Schena et al.** *Science,* 1995, vol. 270, 467
- **Gress, T.M. ; Hoheisel, J.D. ; Lennon, G.G. ; Zehetner, G. ; Lehrach, H.** *Mamm. Genome,* 1992, vol. 3, 609
- **Piétu G. ; Alibert, A. ; Guichard, V. ; Lamy, B. ; Bois, F. et al.** *Genome Research,* 1996, vol. 6, 492
- **DeRisi, J.L. ; Vishwanath, R.Y. ; Brown, P.O.** *Science,* 1997, vol. 278, 680
- **Wodicka, L. ; Dong, H. ; Mittmann, M. ; Ho, M.H. ; Lockhart, D.J.** *Nature Biotechnology,* 1997, vol. 15, 1359
- **B. Jordan.** *Technique de Multiple Messenger Assay,* 1995
- **C. Nguyen ; D. Rocha ; S. Granjeaud ; M. Baldit ; K. Bernard ; P. Naquet ; B. R. Jordan.** Differential gene expression in the murine thymus assayed by quantitative hybridization of arrayed cDNA clones. *Genomics,* 1995, vol. 29, 207-216
- **G. McGall ; J. Labadie ; P. Brock ; G. Wallraff ; T. Nguyen ; W. Hinsberg.** Light-directed synthesis of high-density oligonucleotide arrays using semiconductor photoresists. *Proc Natl Acad Sci U S A,* 1996, vol. 93, 13555-60
- *Journal of Clinical Biology,* Février 2000, vol. 38 (2), 781-788